(19) Europäisches Patentamt / European Patent Office / Office européen des brevets

(11) **EP 3 505 643 A1**

(12) **EUROPEAN PATENT APPLICATION**

(43) Date of publication:
**03.07.2019 Bulletin 2019/27**

(51) Int Cl.:
**C12Q 1/6827** (2018.01)

(21) Application number: **17210908.4**

(22) Date of filing: **28.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **VITO NV**
**2400 Mol (BE)**

(72) Inventors:
• **Hooyberghs, Jef**
  **B-2400 Mol (BE)**

• **Nomidis, Stefanos Konstantinos**
  **B-2400 Mol (BE)**
• **Venken, Tom**
  **2400 Mol (BE)**
• **Carlon, Enrico**
  **3050 Oud-Heverlee (BE)**

(74) Representative: **V.O.**
**P.O. Box 87930**
**Carnegieplein 5**
**2508 DH Den Haag (NL)**

(54) **METHODS FOR DETECTING NUCLEOTIDE VARIANTS**

(57) The present invention relates to a method for determining in a sample solution comprising at least a first target polynucleotide $T_{wt}$, the presence of a low abundant second target polynucleotide $T_M$, said second polynucleotide $T_M$ comprising a second target sequence and differing from a target sequence in one or more nucleotides of said target sequence of said first target polynucleotide $T_{wt}$, wherein the sample contains the first polynucleotide $T_{wt}$ in a concentration C1 which is in excess of a concentration C2 of said second polynucleotide $T_M$ in said sample, said method comprising :
- providing first probes $P_1$ in a first concentration $C_{P1}$, wherein the first probes $P_1$ have a first nucleotide sequence complementary to the first target sequence of the first target polynucleotide $T_{wt}$ for hybridization with that first target sequence,
- providing second probes $P_2$ in a second concentration $C_{P2}$, wherein the second probes $P_2$ have a second nucleotide sequence complementary to the second target sequence of the second target polynucleotide for hybridization with that second target sequence,
- wherein the concentration $C_{P1}$ of the first probes $P_1$ is in excess of the concentration $CP_2$ of the second probes $P_2$, and in excess of the concentration C1 of the first polynucleotide,
- contacting said sample solution with said first and said second probes to cause hybridization with at least the first and second polynucleotides,
- generating a detection hybridization signal from the probes that have been hybridized
- detecting the thus generated signal and the signal intensity ($IT_M$) and analyzing the signal intensity.

Fig. 1

**Description**

The invention relates to methods which allow for the detection of minor concentrations of specific polynucleotide variants, such as mutant genes $T_M$, in a sample comprising an excess concentration of a target polynucleotide, for example a wild type polynucleotide $T_{wt}$, which methods also allow for a reliable identification and/or quantification of such polynucleotide variants in a sample.

Mutations in nuclear genes, which involve the occurrence of particular polymorphic variants at a genetic locus are known to give rise to human inherited diseases and cancers. The mutation may involve a single nucleotide difference, i.e. a point mutation. At the cellular and tissue level, polymorphisms at a specific genetic locus may give rise to significantly altered cellular behavior. Detecting the range of polymorphic variants at a genetic locus and the frequency of their occurrence requires the detection of alleles or variants. Those alleles or variants are potentially present in a low concentration. Even relatively small cell or tissue samples contain millions of DNA molecules with the particular genetic locus, and the wild-type DNA will usually be present in a vast excess over the low abundant mutant DNA. As a result, the detection of such rare mutations presents a tremendous challenge.

WO2014/096126 discloses a method for enriching low abundance alleles (e.g. mutant DNA) to allow for subsequent detection of such alleles, by selective elution of the mutant nucleic acid. The sample containing the variant of the target nucleic acid to be enriched in a low abundance, in the presence of a large excess of the other variant, is hybridized with an oligonucleotide that is complementary to one strand of the target nucleic acid sequence. The oligonucleotide is selected such that it has a mismatch at the single nucleotide position with the variant to be enriched. After having been hybridized, the duplex polynucleotides are contacted with a mismatch intercalating compound comprising an affinity label, capable of binding to the duplex, and subjected to an affinity matrix that recognizes and binds to the affinity label. Thereafter, the buffer which contains one strand of the enriched variant of the target nucleic acid is collected.

US2003/0143584 also discloses method for detecting low frequency mutations in a target sequence from a DNA sample by enriching the DNA sample for one or more target sequences, wherein the enrichment step comprises sequence-specific hybridization to the target sequences with one or more labeled probes, wherein each labeled probe is complementary to a specific target sequence, resulting in about a $10^3$-fold to about a $10^4$-fold enrichment of target sequences from the DNA sample thereby obtaining a target-enriched sample; and detecting mutations in the target sequence or sequences from the target-enriched sample. The enrichment step comprises denaturing double-stranded DNA and contacting it with one or more probes comprising a sequence complementary to one or more target sequences to form probe-fragment hybrid molecules. Isolating the probe-fragment hybrids from the mixture.

In frequently used hybridization-based read-out technologies, one or more ssDNA sequences in a sample solution (targets) are contacted with a collection of one or more ssDNA sequences (probes) immobilized on a surface and grouped in distinguishable spots. A spot is a local space on a microarray slide that contains a large number of identical sequences corresponding to a certain type of probe in the probe set. Therefore, typically, each spot represents a single type of probe. Each of these identical sequences within a spot is supposed to be hybridized to a floating target sequence of the sample solution, depending on the affinity between the two sequences. This affinity is sequence dependent and determines the fraction of hybridized probes in a spot. Hybridisation may take place, regardless whether the probe DNA sequences are fully complementary to the targets or partially complementary. By labelling the targets with a marker, for example a fluorescent dye, the intensity of the radiation or fluorescence emitted by each spot can be measured and is representative for the number or fraction of the probes that has been hybridized.

In most hybridization-on-surface techniques the theoretical basis for determining from a measured signal intensity, the number or fraction of the probe that has been hybridized, is the standard Langmuir isotherm adsorption model. In a hybridisation model with Langmuir isotherm (Hooyberghs et al., Nucleic Acids Res. 2009, 37, e53), the relationship between the detected intensity and the hybridisation affinity for the target-probe hybrid can approximately be written as equation (1) below. Based on this, the total emitted intensity $I_i$ from the i-th spot at equilibrium is found to be

$$I_i = \sum_j \frac{AK_{ij}c_j}{1 + \sum_l K_{il}c_l} \qquad (1)$$

wherein $I_i$ is the detected hybridisation intensity for a spot i, A is a proportionality factor for the intensity a (device-dependent) signal-amplification factor and is e.g. system dependent, and $K_{ij}$ the equilibrium constant. $K_{ij}$ the equilibrium constant is known from thermodynamics to be

$$K_{ij} = exp\left(-\frac{\Delta G_{ij}}{RT}\right) \qquad (2)$$

where R is the gas constant and T the temperature of the system, $\Delta G_{ij}$ is the free energy of hybridization between the i-th probe and the j-th target. $\Delta G_{ij}$ is a sequence dependent measure for the affinity, i.e. the free energy difference between two ssDNA sequences and their DNA duplex formed by the hybridization. R is the ideal gas constant, and T is the experimental temperature.

This equation can be calculated for each spot of the microarray, i.e. for each probe type in a probe set. The denominator in equation (1) accounts for the saturation of the available amount of probes, due to their hybridization with the targets. For small enough target concentrations this effect may be neglected, and the denominator can be approximated by unity, which gives

$$I_i = \sum_j AK_{ij}c_j. \qquad (3)$$

Thereby it is assumed that the hybridisation between the target sequence and probe sequence is in thermodynamic equilibrium, and that the fraction of hybridized probes in a microarray spot is such that the detected intensities are significantly above the background of the detection technique, and far from saturation.

One of the essential approximations of the Langmuir model is that target depletion is neglected, i.e. the decrease of the "available"-target concentration in the sample solution due to the hybridization of the target ssDNA with probes of the micro-array is neglected. In other words, the Langmuir model assumes that the target can be represented by a constant reservoir. This assumption or simplification of the Langmuir model is acceptable provided the number of probes on the micro array surface is significantly smaller than that of the targets in the sample to be analysed. When however this condition is not met, the Langmuir model no longer fits.

Although target depletion has been already considered in at least two studies [Ono, N. et al. Bioinformatics 24, 1278-1285 (2008) and Burden, C. J., & Binder, H. Phys. Biol. 7, 016004 (2009)], to the best of our knowledge it has not been exploited to improve the sensitivity and selectivity of hybridisation-based detection techniques, in particular it has not been exploited to improve the sensitivity and selectivity of hybridisation-based detection techniques focusing on detecting small traces of a mutant DNA in a sample rich in wild type DNA.

The present invention therefore seeks to provide a method which permits to improve the sensitivity and selectivity of hybridisation-based detection techniques, particularly those focusing on detecting small traces of a mutant DNA in a sample rich in wild type DNA.

The present invention relates to methods for analyzing hybridization. More particularly, the methods described herein allow for the detection of the presence of minor concentrations of specific polynucleotide variants such as mutant genes in a sample further comprising a target polynucleotide in excess with respect to the specific polynucleotide variant, for example a wild type polynucleotide $T_{wt}$, and allow a reliable quantification of such variant polynucleotides in the sample, without the need for using a reference sample.

Thereto, the present invention relates to a method for determining in a sample solution comprising at least a first target polynucleotide $T_{wt}$ in the presence of a low abundant second target polynucleotide $T_M$, said second polynucleotide $T_M$ comprising a second target sequence and differing from a target sequence in one or more nucleotides of said target sequence of said first target polynucleotide $T_{wt}$, wherein the sample contains the first polynucleotide $T_{wt}$ in a concentration $C_1$ which is in excess of a concentration $C_2$ of said second polynucleotide $T_M$ in said sample, said method comprising :

- providing first probes $P_1$ in a first concentration $C_{P1}$, wherein the first probes $P_1$ have a first nucleotide sequence complementary to the first target sequence of the first target polynucleotide $T_{wt}$ for hybridization with that first target sequence,
- providing second probes $P_2$ in a second concentration $C_{P2}$, wherein the second probes $P_2$ have a second nucleotide sequence complementary to the second target sequence of the second target polynucleotide for hybridization with that second target sequence,
- wherein the concentration $C_{P1}$ of the first probes $P_1$ is in excess of the concentration $C_{P2}$ of the second probes $P_2$, and in excess of the concentration $C_1$ of the first polynucleotide,
- contacting said sample solution with said first and said second probes to cause hybridization of the said probes with at least the first and second polynucleotides,
- generating a detection signal from the first and second probes that have been hybridized,
- detecting the thus generated signal and in intensity of said signal ($IT_M$) and analyzing the signal intensity.

In the present invention the probe $P_1$, having a nucleotide sequence that is complementary to that of the first target polynucleotide sequence, is present in a concentration which is in substantial excess of the sum of the concentration of the first target polynucleotide $C_1$ . As $C_1$ is in excess of $C_2$, probe $P_1$ is present in a concentration which is in substantial excess of the second target polynucleotide $C_2$ contained in the sample. The probe $P_1$ is further present in a concentration which is in substantial excess of the concentration of any other probes present, for example second probes $P_2$. As a result, when being contacted with the probes, the sample will be depleted in the first target nucleotide and detection of the second target polynucleotide, which otherwise would be masked by the dominant presence of the first target polynucleotide, is made possible. Detection of the second target polynucleotide can now be effectuated even in samples which contain only small traces of the second target polynucleotide. Because the second probes $P_2$ contain a nucleotide sequence that is complementary to the second target nucleotide (often DNA), the second probes will favor hybridization with the second polynucleotide, and give rise to a detection signal.

In a practical example, the first target polynucleotide may correspond to a wild type polynucleotide, and the second target polynucleotide may correspond to a mutant thereof, comprising a mutation in one or more nucleic acids of one ore more nucleotide sequences.

In a preferred embodiment, in addition to first and second probes $P_1$ and $P_2$, third probes $P_3$ are provided in a third concentration $C_{P3}$, wherein the third probes $P_3$ have a third target nucleotide sequence which contains at least one mismatch to the target nucleotide sequence of the first target polynucleotide and at least one mismatch to the target nucleotide sequence of the second target polynucleotide, wherein the concentration $C_{P1}$ of the first probes $P_1$ is in excess of the sum of the concentrations of the second and third probes $P_2$ and $P_3$, and in excess of the concentration C1 of the first target polynucleotide.

The presence of further types of probes in addition to the first and second probes may result in a further reduction of the detection limit of the low abundant target sequence $T_M$. The presence of a third or any further additional probes moreover permits to dispense with the need for analysing a reference sample

According to a preferred embodiment, the first probes $P_1$ are present in an excess concentration $CP_1$ relative to the concentration $CP_2$ of the second probes, wherein the excess is at least a factor 5, preferably at least a factor 10 relative to the concentration $CP_2$ of the second probes. If so desired, the excess may even be higher and for example correspond to a factor 25 or 50 or any other number considered suitable by the skilled person, to minimize the risk to masking of a signal due to the second, for example, mutant polynucleotides by the first target polynucleotide. Also, preferably the excess concentration $C_{P1}$ of the first probes $P_1$ is a number which corresponds at least to the sum of the concentration $CP_2$ of the second probes $P_2$ and the concentration $CP_3$ of the third probes $P_3$, multiplied with a factor of at least 5, preferably at least a factor 10. If so desired, the excess may even be higher and for example correspond to a factor 25 or 50 or any other number considered suitable by the skilled person.

A further preferred embodiment of this invention is characterised in that the concentration $C_{P1}$ of the first probes $P_1$ corresponds to the concentration $C_1$ of the first target sequence of the first target polynucleotide in the sample solution, multiplied with a factor of at least 5, preferably at least a factor 10. If so desired, the excess may even be higher and for example correspond to a factor 25 or 50 or any other number considered suitable by the skilled person to ensure depletion of the target polynucleotide $T_{wt.}$

Another preferred embodiment of this invention is characterised in that the concentration $C_{P2}$ of the second probes $P_2$ is in excess of the concentration $C_2$ of the second polynucleotide. The skilled person will be capable of identifying a suitable excess. Nevertheless, the excess of the concentration of the second probes $P_2$ with respect to the concentration $C_2$ of the second polynucleotide is at least a factor 2, preferably a factor 5, preferably at least a factor 10 relative to the concentration $CP_2$ of the second probes. If so desired, the excess may even be higher and for example correspond to a factor 25 or 50 or any other number considered suitable by the skilled person, to minimize the risk to masking of a signal due to mutant polynucleotides by the target polynucleotide.

In particular embodiments, the methods as envisaged herein further comprise determining the relative amount of said first target polynucleotide $T_{wt}$ and said second target polynucleotide $T_M$ in said sample solution.

In particular embodiments, said sample solution as envisaged herein is prepared by:

- extracting DNA from a sample of interest;
- amplification of a first target polynucleotide and second target polynucleotide $T_M$ thereof contained in said DNA using a pair of primers of which one primer has a phosphate modification at its 5' end, thereby obtaining double stranded DNA; and
- digesting the 5' phosphate modified strands of said double stranded DNA using lambda exonuclease.

A second aspect of the present invention provides a computer program product for performing, when executed on a computing device, a method for determining the presence of a second target polynucleotide, for example a mutant TM of a target polynucleotide in a sample solution according to the methods as envisaged herein, said computer program product being configured for

- receiving hybridisation intensity $I(P_1)m$ for a sample solution with a first probe $P_1$;
- receiving hybridisation intensity $I(P_2)m$ for a sample solution with a second probe $P_2$;

- analysing and comparing $I(P_1)m$ and $I(P_2)m$, preferably determining the ratio $I(P_1)m/I(P_2)m$ and comparing with the ratio $I(P_1)/I(P_2)$, and determining the presence of a second target polynucleotide, for exampla a mutant polynucleotide $T_M$, in said sample solution based thereon.

Another aspect of the present invention relates to a device configured for performing the method for determining the presence of a second target polynucleotide, for example a mutant $T_M$ of a first target polynucleotide in a sample solution as envisaged herein, comprising one or more sets of reaction vessels, feeds for reagents connected thereto and a detection unit and a processing unit comprising the computer program product as envisaged herein.

**BRIEF DESCRIPTION OF THE DRAWINGS**

The following description of the figures of specific embodiments of the methods and instruments described herein is merely exemplary in nature and is not intended to limit the present teachings, their application or uses. Throughout the drawings, corresponding reference numerals indicate like or corresponding parts and features.

Figure 1 shows a schematic representation of the concept of a particular embodiment of the present invention, in particular a schematic representation of the hybridisation between first second and third probes and respectively the first and second target polynucleotide sequences in the sample, with the sample containing both a first, wild type, target polynucleotide and a second target polynucleotide which is a mutant thereof.

Figure 2 shows a schematic representation of the principle of hybridization.

In the description hereafter, whenever two indices are present, the first one refers to the probe and the second one refers to the target.

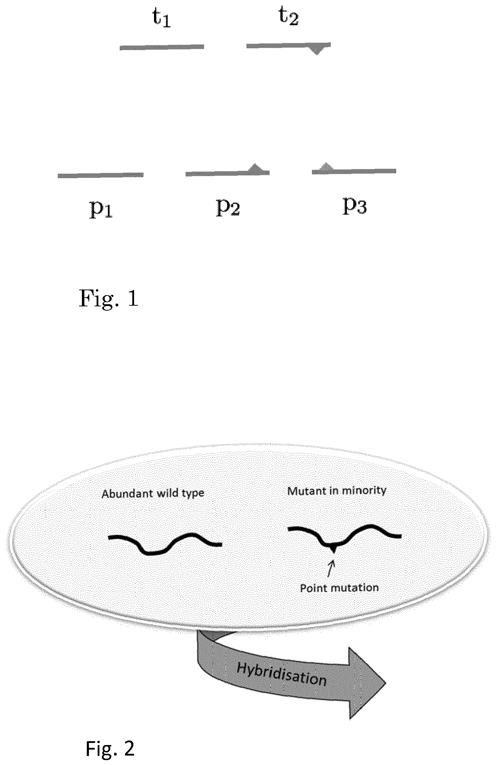

In this context, specific ways to detect and/or identify mutant polynucleotides in a sample solution will be explained below, using theoretical concepts based on the thermodynamics of DNA hybridisation, as illustrated for a particular embodiment in Figure 1. The skilled person will understand that this also applies, mutatis mutandis, to nucleotides other than DNA. While in the following, the theoretical concept is explained in the context of microarray data analysis, the skilled person will understand this also applies for other experimental setups for measuring/analysing hybridisation, including hybridisation in solution.

**DETAILED DESCRIPTION OF THE INVENTION**

While potentially serving as a guide for understanding, any reference signs used herein and in the claims shall not be construed as limiting the scope thereof.

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments described herein are capable of operation in other sequences than described or illustrated herein.

The values as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to ensure one or more of the technical effects envisaged herein. It is to be understood that each value as used herein is itself also specifically, and preferably, disclosed. Typically, the term "about" should be read in this context, in particular in the context of the value of the ratio $I(PA)/I(PB)$.

The recitation of numerical ranges by endpoints includes all numbers and fractions subsumed within the respective ranges, as well as the recited endpoints.

All documents cited in the present specification are hereby incorporated by reference in their entirety.

Unless otherwise defined, all terms used in disclosing the concepts described herein, including technical and scientific terms, have the meaning as commonly understood by one of ordinary skill in the art. By means of further guidance, definitions for the terms used in the description are included to better appreciate the teaching of the present disclosure. The terms or definitions used herein are provided solely to aid in the understanding of the teachings provided herein.

The term "polynucleotide" as used herein may include oligonucleotides and refers to a polymer composed of nucleotide monomers, typically having a length of at least 10 nucleotides. Typically, the polynucleotides such as the target polynucleotides and probes referred to herein are single-stranded polynucleotides. As used herein, the term "polynucleotide" may include deoxyribonucleic acid (DNA), ribonucleic acid (RNA) or peptide nucleic acid (PNA).

The term "equilibrium" as used herein refers to thermodynamic equilibrium and indicates a situation wherein a steady state is obtained such that the number of conventional target-probe bindings does not substantially change over time. The term "non-equilibrium" or "non-equilibrium effects" refers to occurrence of a target-probe binding state that may change over time. The term "free energy" as used herein refers the Gibbs free energy ($\Delta G$) or chemical potential.

In this patent application, the wording "hybridisation" refers to nucleic acid hybridisation. With nucleic acid hybridisation is meant the process of establishing a non-covalent sequence-specific interaction between two or more complementary strands of nucleic acids into a single hybrid. The strands of nucleic acids that may bind to their complement can for example be oligonucleotides, DNA, RNA or PNA. The basic components of the strands of nucleic acids are nucleotides. Hybridisation encompasses both the binding of two perfectly complementary strands (in the Watson-Crick base-pairing senses), as well as binding of non-perfect complementary strands. With a non-perfect complementary strand is meant strands having a small number of non-complementary elements such as one, two or more non-complementary elements, preferably one or two non-complementary elements. In principle there is no limit to the number of non-complementary elements but the higher the number of non-complementary elements, the easier these are detectable.

All documents cited in the present specification are hereby incorporated by reference in their entirety.

Reference throughout this specification to "one embodiment" or "an embodiment" means that a particular feature, structure or characteristic described in connection with the embodiment is included in at least one embodiment envisaged herein. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are also envisaged herein, and form different embodiments, as would be understood by those in the art. For example, in the appended claims, any of the features of the claimed embodiments can be used in any combination.

The term "second target polynucleotide" or "a mutant polynucleotide" or "mutant" ($T_M$) as used herein refers to a polynucleotide having a sequence (the mutant sequence) which differs from the sequence of a certain first target or 'wild type' polynucleotide ($T_{wt}$) in one or more nucleotides. It will be understood by the skilled person that the term "mutant" is not limited to sequences which are the result of a change in the first target polynucleotide in a specific organism, tissue or cell but also include naturally occurring (i.e. evolutionary) sequence variants. More particularly in the context of the present application, these differences or mutations are located within a certain subsequence of the target polynucleotide, referred to herein as the "target sequence". Again, it will be understood that the "first target sequence" is the nucleotide sequence as present in the first type polynucleotide or target wild type polynucleotide and is used as the reference sequence. In particular embodiments, the mutant polynucleotide $T_M$ only differs from the first target or the wild type polynucleotide $T_{wt}$ in one or more nucleotides within the target sequence. Where in this patent application reference is made to first target polynucleotide, this may be equivalent to "wild type" polynucleotide and vice versa. Preferably, the mutant polynucleotide $T_M$ differs from the first target polynucleotide $T_{wt}$ in a limited number of nucleotides within the target sequence, preferably in at most two nucleotides, such as in one or two nucleotides. In particular embodiments, the second target or mutant polynucleotide differs from the first target wildtype polynucleotide in only one nucleotide.

Although the present method focuses on the interaction between a strand that initially is in a sample solution, and a strand that is bound to a surface, it is noted that hybridisation may occur between nucleic acid strands that both are in solution. The strands initially present in the sample solution and of interest for analysis are typically referred to in the art as the "target", whereas the strand which is to hybridize to the target is referred to as "probe".

The mutant polynucleotide and the wild type polynucleotide referred to herein are both potentially present in the sample and may both be considered as "targets". In preferred embodiments both the mutant polynucleotide(s) $T_M$ and target wild type polynucleotide $T_{wt}$ referred to herein are both present in the sample.

The probe may for example be an oligonucleotide, such a DNA, RNA or PNA sequence (partially) complementary to a target. In the methods envisaged herein, the probes may be contacted with the target in the sample by introducing the probes into the sample solution. Preferably, the probe is bound to a surface, such as a carrier, particularly on a microarray. However, in particular embodiments of the present methods the probes are present in solution. Typically, the probes as envisaged for use herein are different (single-stranded) probes having different binding affinities for the (target sequence of the) target polynucleotide $T_{wt}$ and the mutant polynucleotide $T_M$. It will be understood by the skilled person however that the reference to the "target" polynucleotide and the "mutant" polynucleotide is arbitrary in that they can be interchanged.

The probes envisaged for use in the methods provided herein may contain a hybridisation sequence. The hybridisation sequence is the sequence intended for hybridisation with the target sequence and thus its sequence will be determined by the target sequence. Optionally, the probe may further comprise a tail sequence, which may be used to hybridize to other sequences, for tagging of the probe, etc... The length of the hybridisation sequence typically corresponds to the target sequence, i.e. contains the same number of nucleotides. A so-called "perfect match probe" as used herein refers to a probe having a hybridisation sequence which is completely complementary to a target sequence of a target polynucleotide. The term "mismatch probe" as used herein refers to a probe having a hybridisation sequence which is non-complementary to the target sequence. The probe is considered non-complementary as soon as one nucleotide differs from the target sequence of the target polynucleotide. In particular embodiments, the hybridisation sequence of the probe comprises one or more, such as at most two, non-complementary nucleotides with respect to the target sequence in the target polynucleotide.

Provided herein are methods and systems for analyzing hybridisation for determining the presence or detecting of small traces of a second polynucleotide comprising a second sequence, for example a mutant polynucleotide comprising a mutant sequence, in a sample solution which comprises a small trace of the mutant polynucleotide and which is rich in a first polynucleotide for example a target nucleotide, for example the wild-type polynucleotide. Accordingly, in a first aspect, the present application provides methods for determining the presence of a small trace of a second, mutant polynucleotide in a sample solution rich in the first, wild type polynucleotide. Within the scope of this invention, with a small trace is meant a relative amount of the second polynucleotide with respect to the first polynucleotide on weight basis, which is maximum 0.01 %, preferably maximum 0.005 %, more preferable maximum 0.001 % or even below that, It will be clear to the skilled person that some polynucleotides, in particular mutant polynucleotides may be present in a biological sample further comprising the wild type polynucleotide, in extremely small concentrations or weight ratio's.

The methods for determining the presence of a second, for example a mutant polynucleotide $T_M$ in a sample solution further containing an excess of a first polynucleotide $T_{wt}$, as envisaged herein comprise the steps of contacting the sample with at least two different probes which differ in their complementarity to the target and mutant sequence. The present inventors have found that by comparing the hybridisation intensities of hybridisation experiments on a sample solution using specific probes, it is possible to identify mutant polynucleotides in a mixture of mutant $T_M$ and wild type polynucleotide $T_{wt}$, at low concentrations of the second, mutant relative to the first, wild type, with a minimal set of probes without the need of a parallel reference measurement or a reference sample. The methods are based on the observation that the lowest detection limit of the second mutant, i.e. the lowest concentration of a mutant, that may be detected in a sample may be significantly reduced in comparison to prior art techniques, provided the concentration $C_{P1}$ of the first probes $P_1$ is

- in excess of the concentration $C_{P2}$ of the second probes $P_2$,
- and in excess of the concentration $C_1$ of the target sequence of the target polynucleotide.

In a preferred embodiment further the concentration $C_{P2}$ of the second probes $P_2$ may be in excess of the concentration $C_2$ of the second sequence of the second polynucleotide.

Indeed, provided these conditions are met, the detection limit of a second target low abundant polynucleotide $T_M$ in a sample containing an excess of a first target polynucleotide $T_{wt}$ can be significantly enhanced.

In a preferred embodiment, the methods of the present invention are based on the provision of suitable probes. In particular the methods encompass the step of designing and/or selecting probes, designed such that each probe (and more particularly the hybridisation sequence of the probe) contains a (different) level of mismatch with the wild type target sequence.

More particularly, the methods provided in the present invention comprise the steps of:

(i) providing a probe pair consisting of a first probe $P_1$ and a second probe $P_2$ different from $P_1$;
(ii) contacting a sample solution with said first probe $P_1$ and said second probe $P_2$, and obtaining or detecting hybridisation intensities $I(P_1)m$ and $I(P_2)m$ for said first probe $P_1$ and said second probe $P_2$ of said probe pair, respectively; and
(iii) analysing and comparing $I(P_1)m$ and $I(P_2)m$ and determining the presence of said mutant polynucleotide $T_M$ in said sample solution based thereon.

More particularly, in these methods a first probe $P_1$ and a second (different) probe $P_2$ are designed or selected such that the hybridisation sequences of said first probe $P_1$ and second probe $P_2$ are characterized in that

(i) the first probe $P_1$ is fully complementary to the nucleotide sequence of the first target sequence, present in an excess concentration in the sample solution,

(ii) the second probe $P_2$ is fully complementary to the nucleotide sequence of the second or mutant target sequence, present in low abundance in the sample solution, and the second probe $P_2$ comprises at least one non-complementary nucleotide with respect to the first target sequence of the first polynucleotide or wild type.

A further preferred embodiment is characterised in that the third probe $P_3$ comprises at least one non-complementary nucleotide with respect to the mutant sequence. In particular, the at least one non-complementary nucleotide of probe $P_3$ (vs the mutant sequence of $T_M$) is different from the non-complementary nucleotide of probe $P_2$ (vs the target sequence of $T_{wt}$).

As used herein, probe $P_2$ is also referred to as the "mutant probe", and $P_1$ is referred to as the "wild type probe".

In certain embodiments, probe $P_2$, and optionally also probe $P_1$, is designed using a theoretical model for $\Delta G$ calculation, preferably using a Nearest Neighbor model ((Bloomfield VA et al., "Nucleic Acids Structures, Properties and Functions, University Science Books, Mill Valley, 2000). This is possible since a stringent relation (e.g. the Langmuir isotherm under certain experimental conditions) exists between measured intensities I and the corresponding free energy $\Delta G$. Nearest neighbor models provide a good estimation of the hybridisation free energy as a sum of dinucleotide parameters. In these embodiments, the method may comprise calculating or estimating the hybridisation free energy for the hybridisation between the wild type target polynucleotide Twt and probe $P_1$; followed by estimating or calculating the hybridisation free energy for the hybridisation between the wild type target polynucleotide Twt and a plurality of candidate probes $P_2$ based on a Nearest-Neighbor model. In particular embodiments, probe $P_2$ is selected following this theoretical calculation as the probe for which $\Delta G(T_{wt},P_2)$ minus $\Delta G(T_{wt},P_1)$ is minimal, more preferably as the probe for which $\Delta G(T_{wt},P_2)$ is about equal to $\Delta G(T_{wt},P_1)$ (or wherein $\Delta G(T_{wt},P_2)$ minus $\Delta G(T_{wt},P_1)$ is about 0). It is an advantage of embodiments according to the present invention that hybridisation free energy can be determined accurately for hybridisation between a target initially in solution and a probe bound to a surface, such as for example may occur in microarrays. Advantageously, using a theoretical model is a quick and straightforward way to design probe $P_2$. It will be understood that in what follows when referring to the nature of the probes, more particularly to their ability to hybridize with a target sequence, this in fact refers to the hybridisation sequence of the probes.

In certain embodiments, probe $P_2$ can be designed to be specific for the mutant polynucleotide $T_M$, i.e. comprising a nucleotide complementary to the one or more nucleotides differing between the sequence of the wild type and the mutant polynucleotides, but comprises in addition one or more nucleotides non-complementary to the target wild type polynucleotide $T_{wt}$.

The methods as envisaged herein are of particular interest for the analysis of a sample solution which comprises a mixture of the target polynucleotide [$T_{wt}$ or wild type (wt)] and a mutant polynucleotide [$T_M$ or (mut)]. More particular they are of interest in samples which are characterized in that the concentration of the mutant polynucleotide [Ci] is significantly smaller than the concentration of the target or wild type polynucleotide [$C_2$]. The ratio of these concentrations [$C_2/C_1$] is also referred to herein as the "relative concentration" of mutant polynucleotide in the sample solution. In preferred embodiments, the relative concentration of mutant polynucleotide in the sample solution is between 0.001 and 0.5, more preferably between 0.001 and 0.1. The relative concentration may also be expressed as a percentage, which refers to $100*[C_2/C_1]$.

The sample solution may be prepared for use in the methods envisaged herein using standard methods known in the art. This may include extracting DNA or other polynucleotides from a sample of interest, followed by amplification of certain fragments within the extracted DNA. Typically, amplification is performed using PCR (polymerase chain reaction). However, this results in double stranded DNA, whereas single-stranded DNA is preferred for the present methods. Indeed, hybridisation of double-stranded DNA with nucleic acid probes is hampered by competition between the complementary non-target strand and the probe. Such competition can be avoided by degradation of the complementary strands, for example using lambda exonuclease. Lambda exonuclease is a processive enzyme that acts in the 5' to 3' direction, catalyzing the removal of 5' mononucleotides from duplex DNA. The preferred substrate is 5'-phosphorylated double stranded DNA. Accordingly, in certain embodiments, the preparation of the sample solution may comprise the steps of:

- extracting DNA from a sample of interest;
- amplification of a target polynucleotide and mutants thereof contained in said DNA using a pair of primers of which one primer has a phosphate modification at its 5' end; thereby obtaining double stranded DNA; and
- digesting the 5' phosphate modified strands of said double stranded DNA using lambda exonuclease.

The contacting of the sample with the probes is typically performed under conditions suitable for hybridisation of the target and mutant polynucleotides to said probes. The skilled person understands that relevant parameters for optimizing hybridisation include hybridisation time, temperature, and probe length. In preferred embodiments, the probes have a length ranging from about 20 to about 30 nucleotides. Furthermore, it is preferred that the hybridisation experiments are performed under such conditions that hybridisation has reached equilibrium, e.g. by selecting suitable probe lengths, temperatures, and hybridisation time. A method for determining for which probes or spots the hybridisation has reached equilibrium is described in international patent application WO2011/035801, which is hereby incorporated by reference in its entirety.

Although the methods envisaged herein may be carried out by using probes which are added to the sample in solution, it is preferred that the probes are provided on a surface. The probes may be provided on any type of carrier, such as magnetic beads or fibers. However, it is preferred that the probes are provided on a microarray. Thus, in particular embodiments of the methods described herein, the first and second probe of the probe pair are provided on separate spots of a microarray. A microarray as a hybridisation platform contains a large number of probes which are immobilized on a solid surface. Preferably, the probes are provided in spatially separated spots, wherein each spot comprises one (and only one) type of probe. Typically, each spot comprises only a few picomoles of each probe. Typical microarrays comprise hundreds or even thousands of spots. A plurality of microarray platforms suitable for use in the present methods are commercially available, and include but are not limited to the platform provided by Agilent, the GeneChips platform from Affymetrix or CodeLink Bioarray platform from Amersham Biosciences.

In preferred embodiments, the first and second probes, and preferably also the third probes $P_3$ may be provided on the same microarray. This can facilitate comparing the hybridisation intensities for the probe pair.

The hybridisation intensity I as envisaged herein is a value representing the fraction of a certain probe which is hybridized. In the methods described herein, detection of hybridisation intensity may be performed using a marker associated with the formed hybrid, such as for example a fluorescence marker or a radio-active marker, or other markers known in the art. In preferred embodiments, the marker used for probe $P_1$ is the same as the marker used for probe $P_B$. However, this is not critical for the present methods. Accordingly, different markers may be used for probe $P_1$ and probe $P_2$ of the probe pair. In certain embodiments, the detection of the hybridisation intensity may be performed using a label-free method as known by the skilled person, such as surface-enhanced Raman spectroscopy.

Typically in hybridisation experiments, intensity of the radiation or fluorescence provided by the markers is detected and representative for the number of hybrids formed. Thus, in certain embodiments, the hybridisation intensities may be induced by emission of a label associated with a hybrid formed by binding of the target polynucleotide or mutant thereof and said probes. Suitable fluorescence markers for the present methods include, but are not limited to, Cy3 and Cy5, which are dyes of the cyanine dye family.

The markers or labels may be associated to the target or mutant polynucleotide prior to or after hybridisation, such as during PCR amplification of the sample DNA. In some embodiments, a fluorescent dye or other marker compound may be associated directly to the target or mutant thereof. In other embodiments, the marker compounds may be associated to the target or mutant thereof in an indirect manner, for example via a "barcode", which is a strand having a hybridisation sequence which is complementary to a tail sequence which is present on the mutant polynucleotide of interest and on the target polynucleotide, thereby allowing hybridisation between the barcode and target (or mutant thereof), and therefore indirect coupling of the fluorescence marker or other marker to the target. More particularly, the strand hybridizes to a tail sequence outside the target sequence of the target polynucleotide, such that it does not significantly interfere with the hybridisation between the targets and the probes.

After the detection of the signal resulting from the hybridisation of the sample DNA with the probes, the analysis is performed. In this context, specific ways to detect and/or identify mutant polynucleotides in a sample solution will be explained below, using theoretical concepts based on the thermodynamics of DNA hybridisation, as illustrated for a particular embodiment in Figure 1. The skilled person will understand that this also applies, mutatis mutandis, to nucleotides other than DNA. While in the following, the theoretical concept is explained in the context of microarray data analysis, the skilled person will understand this also applies for other experimental setups for measuring/analysing hybridisation, including hybridisation in solution.

In microarray data analysis, each probe intensity , which is representative for the hybridisation intensity, is associated to a signal from a spot. A spot is a local space on the microarray slide that contains a large number of identical sequences corresponding to a certain type of probe in the probe set. Therefore, typically, each spot represents a single type of probe. Each of these identical sequences within a spot is supposed to be hybridized to a floating target sequence depending on the affinity between the two sequences. This affinity is sequence dependent and determines the fraction of hybridized probes in a spot.

In a hybridisation model with Langmuir isotherm (Hooyberghs et al., Nucleic Acids Res. 2009, 37, e53), the relationship between the detected intensity and the hybridisation affinity for the target-probe hybrid can approximately be written as equation (1) (assuming the hybridisation between the target and probe sequences are in thermodynamic equilibrium, and that the fraction of hybridized probes in a microarray spot is such that the detected intensities are significantly above the background yet far from saturation):

$$I = A.e^{\wedge}(-\Delta G/RT)$$

wherein I is the detected hybridisation intensity, A is a proportionality factor for the intensity and is e.g. system dependent, $\Delta$G is hybridisation free energy as a sequence dependent measure for the affinity, i.e. the free energy difference between two ssDNA sequences and their DNA duplex formed by the hybridisation, R is the ideal gas constant, and T is the experimental temperature.

This equation can be calculated for each spot of the microarray, i.e. for each probe type in a probe set.

In case of the hybridisation between a sample containing either a first target or wild type sequence ($T_{wt}$) or either a mixture of a target sequence $T_{wt}$ and a mutant sequence $T_M$ and a probe pair, $P_1$ and $P_2$, wherein probe $P_2$ comprises a sequence which is either fully or partly complementary to the mutant sequence $T_M$ and wherein probe $P_1$ comprises a sequence which is fully or partly complementary to the wild type target sequence $T_{wt}$, the following relations can be deduced.

The proposed method is based on an extended version of the Langmuir adsorption theory, which takes account of the depletion of the target polynucleotide in the sample, following its hybridization with the first probe. It has now been observed that with the methods of this invention, in the analytical methods used for detecting the hybridisation, the detection of a second, low-abundant target can be significantly enhanced.

The principle of the present invention may be presented as shown in figure 1. A sample solution contains a first target or wild type polynucleotide t1 (wild type) and a second or mutant polynucleotide t2 (mutant), wherein the latter for example contains a single point mutation represented by the triangle, compared to the wild type polynucleotide t1. In the sample solution, the wild type polynucleotide t1 is present in high abundancy, whereas the mutant polynucleotide t2 is present in low abundancy when compared to the wild type t1. Whereas probe $P_1$ is complementary, and preferably fully complementary to wild type polynucleotide t1, probe $P_2$ is complementary, and preferably fully complementary to mutant polynucleotide t2. Probe $P_3$ contains one mismatch with respect to wild type t1, and two mismatches, compared to wild type t1 and mutant t2.

The method of this invention is carried out in such a way that a strong depletion of the abundant wild-type polynucleotide is achieved. This can for example be achieved by including probes $P_1$ with a probe sequence fully-complementary to that target, wherein the concentration or number of probes $P_1$ is at significantly higher than required to hybridise all of the target wild type polynucleotide, and significantly higher than the concentration of the other probes. As a result a strong depletion of the wild-type in the sample solution is achieved, and the detection of very small traces of mutant is made possible, which would otherwise be masked by the dominant presence of the wild type.

In the methods of this invention preferably a probe sequence $P_2$ is present that is complementary to , and preferably fully-complementary to the mutant. As it hybridizes very favorably with the latter, a corresponding hybridisation signal is generated.

The principle outlined above may be represented by the following approximations:

The concentration of first probes $P_1$ is significantly larger than the concentration of the second probes $P_2$, and in the preferred embodiment containing also a third probes $P_3$ the concentration of $P_1$ is significantly larger than the concentration of the second probes P2 and the concentration of the third probes $P_3$:

$$a_1 \gg a_{2,3} \frac{K_{PM}}{K_{1MM}}$$

As both the first and second target polnucleotides, i.e. the wild type and mutant, are strongly depleted when being contacted with probes $P_1$ and $P_2$, or preferably also with probes $P_3$, a lower limit in a1 can be defined as follows :

$$a_1 \gg \frac{1}{K_{1MM}}$$

In the present invention, all probes remain far from saturation or in other words a significant number of probes $P_1$, $P_2$ and preferably $P_3$ remain which have not been hybridized. As a result an upper limit for the concentration c1 of wild type polynucleotide may be identified as follows:

$$c_1 \ll a_1$$

In the above, the parameters K may be calculated from hybridization models ; parameters c and a are design parameters, which may be controlled in the experimental setup in such a way that the conditions outlined above in relation to depletion of the mutant and wild type and the concentration of probes P1, P2, P3 relative thereto, are met.

A preferred embodiment of this invention relates to the design of probes P3 and any other additional probes that may be incorporated. In the example described below, a third probe P3 has been chosen that contained one and two mismatches with respect to the wild-type and mutant DNA, respectively.

In the example described below, to simplify the system, additionally the energetic cost of the one mismatch was assumed to be approximately the same for different DNA duplexes, which is a good approximation for an appropriate selection of the mismatches. It is emphasized that these features contribute to the simplification of the method, much more than to the effectiveness of the method, and the sensitivity of the method of this invention seems not critically affected by different choices of probes and mismatches.

The invention is based on a refined adsorption model, which accounts for the decreasing concentration of the "available" target. If one denotes by $\theta ij$ the fraction of probes at spot i, which have hybridized with the target j, it will obey the following equation :

$$\frac{d\theta_{ij}}{dt} = k_{ij}\left(1 - \sum_l \theta_{il}\right)\left(c_j - \sum_l a_l \theta_{lj}\right) - k_{-ij}\theta_{ij}. \qquad (4)$$

Wherein $k_{ij}$ and $k_{-ij}$ are the hybridization and denaturation rates, respectively. The ratio of the two is $K_{ij} = k_{ij}/k_{-ij}$ , i.e. the equilibrium constant introduced in Eq. (2). $cj$ is the concentration of target $j$, while $ai$ is the concentration of probes at each site $i$, which can be engineered if needed.

In the description hereafter it is assumed that hybridization is assessed using measurement of the fluorescence intensity. It may however be clear to the skilled person that any other conventional hybridization measurement technique may be used. The total fluorescence intensity $li$ emitted by each probe site may be assessed experimentally :

$$I_i = A \sum_j \theta_{ij}, \qquad (5)$$

Herein, A is a device-dependent constant factor, which defines the scale of the fluorescence intensity. The standard Langmuir adsorption model can be obtained from Eq. (4) by setting aj=0, which has the effect that depletion of the target polynucleotide is neglected.

In formula (4), with the probes being far from being saturated, or in other words with a significant number of probes not being hybridized, i.e. when $\sum_l \theta_{il} \ll 1$. :

$$d\theta ij/dt=0$$

This leads to a set of equations in matrix form, which can be inverted using the Sherman-Morrison, as follows :

$$I_i = \sum_j A K_{ij} \widetilde{c}_j \qquad (6)$$

This equation has the same form as equation (3), with the concentrations of the target polynucleotides effectively rescaled according to

$$\widetilde{c}_i = \frac{c_i}{1 + \sum_j a_j K_{ji}} \qquad (7)$$

Further in the example illustrating the present invention, as represented by see Figure 1, the sample contains (1) a first target polynucleotide $T_{wt}$ or t1, preferably the abundant wild-type DNA, in a first concentration c1, and (2) a low abundant second target polynucleotide $T_M$ or $t_2$ which differs from the wild type by a single base, and is present in a concentration $c_2$. In addition, $c_1 \gg c_2$. Hybridisation is carried out by contacting the polynucleotides with three probes $p_1$, $p_2$ and $p_3$ as described above, and for example immobilized on a surface. With $p_1$ fully complementary to target $t_1$ and $P_2$ fully complementary to target $t_2$, and $p_3$ containing one mismatch with respect to t1 and two mismatches with respect to $t_2$. For ease of calculation, it is assumed that $K_{11} = K_{22} = K_{PM}$, $K_{12} = K_{13} = K_{21} = K_{1MM}$ and $K_{21} = K_{2MM}$, which means that it is assumed that all perfect matches have the same hybridization equilibrium constant $K_{PM}$, and that all the single mismatches have the same hybridization equilibrium constant $K_{1MM}$.

From equation (2), it follows that $K_{PM} \gg K_{1MM} \gg K_{2MM}$, i.e that the equilibrium constant in a hybridisation with a perfect match, is higher than the equilibrium constant in a hybridisation involving one mismatch, which in turn is higher than the equilibrium constant in a hybridisation involving two mismatches.

When assessing the hybridization intensity, signals are generated, the intensity of which can be measured and a detection signal S can be defined as follows :

$$ S = \log \ (I_2/I_3) \qquad (8) $$

S contains the ratio of the intensities emitted by P2 and P3. Using the above simplifications, this gives :

$$ S = log \frac{\widetilde{c_1} K_{1MM} + \widetilde{c_2} K_{PM}}{\widetilde{c_1} K_{1MM} + \widetilde{c_2} K_{2MM}} \qquad (9) $$

where the effective concentrations of $t_1$ and $t_2$ are

$$ \widetilde{c_1} = \frac{c_1}{1 + a_1 K_{PM} + a_2 K_{1MM} + a_3 K_{1MM}}, \qquad \widetilde{c_2} = \frac{c_2}{1 + a_1 K_{1MM} + a_2 K_{PM} + a_3 K_{2MM}}. \qquad (9) $$

As a result, in the absence of the second target polynucleotide $\left( c_2 = \widetilde{c_2} = 0 \right)$ the detection signal S will vanish.

In the situation with a sample hybridization where there is no depletion of the first target polynucleotide (wild type), and the sample contains a second target polynucleotide (mutant) which corresponds to the state-of-the art situation : $c_1 K_{1MM} \gg c_2 K_{2MM}$, and the signal is

$$ S_0 \approx log \left( 1 + \frac{c_2}{c_1} \frac{K_{PM}}{K_{1MM}} \right). \qquad (10) $$

Thus, the presence of a second target polynucleotide will lead to a detectable increase in $S_0$. When plugging in equation (10) and solving for $c_2/c_1$ :

$$ \frac{c_2}{c_1} = \left( e^{\sqrt{2}\delta\varepsilon} - 1 \right) exp \left( \frac{\Delta\Delta G_{1MM}}{RT} \right). \qquad (14) $$

By analyzing typical microarray data [e.g. Hooyberghs, Jef, et al. Phys. Rev. E 81, 012901 (2010)], an error of $\delta\varepsilon \approx 0.2$ can be identified. Moreover, taking a temperature of $T$ = 65°C and a representative value $\Delta\Delta G_{1MM}$ = -2.5 *kcal/mol* for the energetic loss of one mismatch [see e.g. Hooyberghs, J. et al. Nucleic Acids Res. 37, e53 (2009)],

$$ \frac{c_2}{c_1} \approx 0.8\%, \qquad (15) $$

in good agreement with the reported value of 1% from a somewhat different microarray approach [Hooyberghs, J. et al. Biosens. Bioelectron. 26, 1692-1695 (2010)].

In the situation where the concentration of the probes P1 is selected such that strong depletion of the first target polynucleotide (wild type) is achieved according to the current invention,

$$ a_1 K_{1MM} \gg a_{2,3} K_{PM}, $$

i.e. the concentration of $p_1$ is so large that its depleting effect of the wild type dominates over $p_2$ and $p_3$, causing hybridization of the second target polynucleotide (mutant) present in a small concentration with respect to the wild type. Equation (9) then leads to

$$\widetilde{c_1} \approx \frac{c_1}{a_1 K_{PM}}, \qquad\qquad \widetilde{c_2} \approx \frac{c_2}{a_1 K_{1MM}}, \qquad\qquad (11)$$

Which take account of the limit of strong depletion, i.e. $a_1 K_{1MM} \gg 1$. Plugging this into Eq. (8) and using again the fact that $c_1 \gg c_2$, yields

$$S \approx log\left[1 + \frac{c_2}{c_1}\left(\frac{K_{PM}}{K_{1MM}}\right)^2\right]. \qquad\qquad (12)$$

Plugging in Eq. (12) and solving again for $c_2/c_1$ gives the expression

$$\frac{c_2}{c_1} = \left(e^{\sqrt{2}\delta\varepsilon} - 1\right)exp\left(2\frac{\Delta\Delta G_{1MM}}{RT}\right). \qquad\qquad (16)$$

The difference between equation (14) and (16) is the additional factor of 2 in the exponent. Using the same representative values for the parameters as above gives

$$\frac{c_2}{c_1} \approx 0.002\%. \qquad\qquad (17)$$

Comparison between Eqs. (15) and (17) reveals that the lowest detection limit for the second target nucleotide (mutant) is now a factor of 400 lower than in the prior art situation where no account is taken of depletion of the first and second target nucleotide.

It will be clear to the skilled person that depending on the nature and number of the target nucleotides, the nature and number of the different probes, a different lowest detection limit may be obtained and that the lowest detection limit may be further reduced.

In certain embodiments, the present methods may comprise determining which of a plurality of candidate mutant polynucleotides is present in the sample solution. In this context, the method comprises contacting the sample solution with a plurality of probes as envisaged herein, wherein a probe P2 specific for one of the candidate mutant polynucleotides and a probe P1 designed or selected as above.

Further provided herein is a computer program product for performing, when executed on a computing device, at least a part of a method for determining the presence of a mutant of a target polynucleotide as described herein. For example, the computer programs may be configured for receiving and analyzing hybridisation intensities according to the methods described herein. For example, the computer program may be configured to analyse the intensity of the hybridisation of the sample solution with a first and second probe of a probe pair, and to statistically analyse and compare the ratio of the hybridisation intensities with the first and second probe as described herein. In particular embodiments, the computer program product is configured for receiving a hybridisation intensity for a sample solution with a first probe PA of a probe pair, for receiving a hybridisation intensity for a sample solution with a second probe PB of said probe pair and analyzing the ratio of the measured intensities and comparing it to the (theoretical) ratio of the hybridisation intensities of the wild type polynucleotide Twt with probes PA and PB, calculated using e.g. a Nearest Neighbor model.

The software may further be configured to perform a statistical analysis of the hybridisation intensity data using a first and second probe of one or more probe pairs in order to determine which of a plurality of candidate mutants is present in a sample solution. In certain embodiments, the computer programs may further be configured for designing suitable probe sets based on information of the target sequence and/or mutations thereof, particularly based on a Nearest Neighbor model for determining the difference in free energy.

In case of implementation or partly implementation as software, such software may be adapted to run on suitable computer or computer platform, based on one or more processors. The software may be adapted for use with any suitable operating system. The computing means may comprise a processing means or processor for processing data.

A further tool provided herein is a device configured for carrying out the methods provided herein. More particularly the device comprises the combination of the necessary hardware and software for carrying out the different steps of these methods. The device may comprise hardware, in the form of reaction vessels and feeds for reagents connected thereto and a detection unit, which can ensure the contacting a sample solution with a first probe P1 and a second probe P2, and hybridisation of the sample solution with said probe P1 and said probe P2. Moreover, the device comprises a processing unit provided with the necessary software for performing the analysis step involving the comparison of the intensity measurements with the first and second probe of a probe pair and optionally a display unit to present the results of said analysis to a user. In particular embodiments, the results are displayed as information on the presence of a mutant polynucleotide in the sample solution.

**Claims**

1. A method for determining in a sample solution comprising at least a first target polynucleotide $T_{wt}$, the presence of a low abundant second target polynucleotide $T_M$, said second polynucleotide $T_M$ comprising a second target sequence and differing from a first target sequence in one or more nucleotides of said first target sequence of said first target polynucleotide $T_{wt}$, wherein the sample contains the first polynucleotide $T_{wt}$ in a concentration $C_1$ which is in excess of a concentration $C_2$ of said second polynucleotide $T_M$ in said sample, said method comprising :

   - providing first probes $P_1$ in a first concentration $C_{P1}$, wherein the first probes $P_1$ have a first nucleotide sequence complementary to the first target sequence of the first target polynucleotide $T_{wt}$ for hybridization with that first target sequence,
   - providing second probes $P_2$ in a second concentration $C_{P2}$, wherein the second probes $P_2$ have a second nucleotide sequence complementary to the second target sequence of the second target polynucleotide $T_M$ for hybridization with that second target sequence,
   - wherein the concentration $C_{P1}$ of the first probes $P_1$ is in excess of the concentration $CP_2$ of the second probes $P_2$, and in excess of the concentration $C_1$ of the first polynucleotide,
   - contacting said sample solution with said first and said second probes $P_1$, $P_2$ to cause hybridization thereof with at least the first and second polynucleotides $T_{wt}$ and $T_M$ of the sample solution,
   - generating a detection hybridization signal from the first and second probes that have been hybridized
   - detecting the thus generated signal and an intensity $IT_{wt}$ and $IT_M$ of the thus generated signal and analyzing the signal intensity.

2. A method according to claim 1, wherein third probes $P_3$ are provided in a third concentration $C_{P3}$, wherein the third probes $P_3$ have a third target nucleotide sequence which contains at least one mismatch to the first target nucleotide sequence of the first target polynucleotide and at least one mismatch to the second target nucleotide sequence of the second target polynucleotide, wherein the concentration $C_{P1}$ of the first probes $P_1$ is in excess of the sum of the concentrations of the second and third probes $P_2$ and $P_3$, and in excess of the concentration $C1$ of the first target polynucleotide.

3. A method according to claim 1 or 2, wherein the first probes $P_1$ are present in an excess concentration $C_{P1}$ relative to the concentration $C_{P2}$ of the second probes, which is at least a factor 5, preferably at least a factor 10 relative to the concentration $C_{P2}$ of the second probes.

4. A method according to claim 2 or 3, wherein the excess concentration $C_{P1}$ of the first probes $P_1$ corresponds to the sum of the concentration $C_{P2}$ of the second probes $P_2$ and the concentration $C_{P3}$ of the third probes $P_3$ multiplied with a factor of at least 5, preferably at least a factor 10.

5. A method according to any of the previous claims, wherein the concentration $C_{P1}$ of the first probes $P_1$ corresponds to the concentration $C_1$ of the target sequence of the target polynucleotide in the sample solution, multiplied with a factor of at least 5, preferably at least a factor 10.

6. A method according to any of the previous claims, wherein the concentration $C_{P2}$ of the second probes $P_2$ is in excess of the concentration $C_2$ of the second polynucleotide, wherein preferably the concentration of the second probes $P_2$ corresponds to a value of at least the concentration $C_2$ of the second polynucleotide multiplied with a factor 2, preferably a factor 5, preferably a factor 10.

7. A method according to any of the previous claims, wherein , wherein the nucleotide sequence of the first probes $P_1$ is fully complementary to the first target sequence of the first target polynucleotide $T_{wt}$.

8. A method according to any one of the previous claims, wherein the nucleotide sequence of the second probes $P_2$ is fully complementary to the second target sequence of the second polynucleotide $T_M$.

9. A method according to any one of the previous claims, wherein the relative concentration of the second target polynucleotide with respect to the first target polynucleotide in the sample solution is between 0.001 and 0.5, more preferably between 0.001 and 0.1.

10. The method according to any one of the preceding claims, further comprising determining the concentration of second polynucleotide $C_2$ in said sample solution from the signal intensity $IT_M$.

11. The method according to any of the previous claims, wherein the detection signal intensities ($IT_{TM}$, $IT_{wt}$) are induced by emission of a label associated with a hybrid formed by binding of said second target polynucleotide to respectively said second probes.

12. The method according to claim 11, wherein probe $P_1$ and probe $P_2$, and preferably probe $P_3$, are differently labelled.

13. A method according to any of the previous claims, wherein the concentration of the first probes $P_1$ and the concentration of the second probes $P_2$ is selected such that after hybridization of the first probes $P_1$ with the first target sequence of the first target polynucleotide $T_{wt}$, and hybridization of the second probes $P_2$ with the second target sequence of the second type polynucleotide $T_M$, a number of first probes $P_1$ and a number of second probes $P_2$ are not hybridized.

14. A computer program product for performing, when executed on a computing device, a method for determining the presence of a mutant $T_M$ of a target polynucleotide $T_{wt}$ in a sample solution according to any one of claims 1 to 13, said computer program product being configured for :

   - receiving hybridisation intensity $I(P_1)m$ for a sample solution with a first probe $P_1$;
   - receiving hybridisation intensity $I(P_2)m$ for a sample solution with a second probe $P_2$;
   - analysing and comparing $I(P_1)m$ and $I(P_2)m$, preferably determining the ratio $I(P_2)m/I(P_1)m$, and determining the presence of a mutant polynucleotide in said sample solution based thereon,

   wherein the first probe $P_1$ comprises at least one non-complementary nucleotide (NT1) with respect to the target sequence of $T_{wt}$ and the second probe $P_2$ comprises at least one different non-complementary nucleotide (NT2) with respect to the target sequence of $T_{wt}$; and wherein the ratio of the hybridisation intensity $[I(P_1)]$ for the hybridisation between the target polynucleotide $T_M$ and the first probe $P_1$ to the hybridisation intensity $[I(P2)]$ for the hybridisation between the target polynucleotide $T_M$ and the second probe $P_2$ is a known value.

15. A device configured for performing the method for determining the presence of a mutant of a target polynucleotide in a sample solution according to any one of claims 1 to 13, comprising one or more sets of reaction vessels, feeds for reagents connected thereto and a detection unit and a processing unit comprising the computer program product according to claim 14.

$$t_1 \qquad t_2$$

$$p_1 \qquad p_2 \qquad p_3$$

Fig. 1

Abundant wild type

Mutant in minority

Point mutation

Hybridisation

Fig. 2

Europäisches
Patentamt

European
Patent Office

Office européen
des brevets

# EUROPEAN SEARCH REPORT

**Application Number**

EP 17 21 0908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 98/24933 A1 (BOSTON PROBES INC [US]; DAKO AS [DK]; COULL JAMES M [US]; HYLDIG NIELS) 11 June 1998 (1998-06-11)<br>* Fig. 1;<br>pages 31-34 "Experiment A" * | 1-15 | INV.<br>C12Q1/6827 |
| X | WO 00/31298 A1 (EXACT LAB INC [US]) 2 June 2000 (2000-06-02)<br>* page 5, line 23 - page 6, line 22; claims 1, 3 and 4 * | 1-15 | |
| A,D | WO 2011/035801 A1 (VITO NV VLAAMSE INSTELLING VOOR TECHNOLOGISCH ONDERZOEK [BE]; UNIV LEU) 31 March 2011 (2011-03-31)<br>* abstract;<br>Fig. 1 * | 1-15 | |
| A | HOOYBERGHS J ET AL: "Hybridisation thermodynamic parameters allow accurate detection of point mutations with DNA microarrays",<br>BIOSENSORS AND BIOELECTRONICS, ELSEVIER BV, NL,<br>vol. 26, no. 4,<br>15 December 2010 (2010-12-15), pages 1692-1695, XP027546903,<br>ISSN: 0956-5663<br>[retrieved on 2010-08-16]<br>* the whole document * | 1-15 | TECHNICAL FIELDS SEARCHED (IPC)<br><br>C12Q |
| A | JEAN-CHARLES WALTER ET AL: "Nonequilibrium effects in DNA microarrays: a multiplatform study",<br>ARXIV.ORG, CORNELL UNIVERSITY LIBRARY, 201 OLIN LIBRARY CORNELL UNIVERSITY ITHACA, NY 14853,<br>8 July 2011 (2011-07-08), XP080514458,<br>DOI: 10.1021/JP2014034<br>* the whole document * | 1-15 | |

The present search report has been drawn up for all claims

| Place of search | Date of completion of the search | Examiner |
|---|---|---|
| Munich | 10 April 2018 | Leber, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 21 0908

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2018

| Patent document cited in search report | | Publication date | Patent family member(s) | | | Publication date |
|---|---|---|---|---|---|---|
| WO 9824933 | A1 | 11-06-1998 | AU | 7625998 | A | 29-06-1998 |
| | | | DE | 69706315 | D1 | 27-09-2001 |
| | | | DE | 69706315 | T2 | 08-05-2002 |
| | | | DK | 0946750 | T3 | 17-12-2001 |
| | | | EP | 0946750 | A1 | 06-10-1999 |
| | | | US | 6110676 | A | 29-08-2000 |
| | | | US | 2003124521 | A1 | 03-07-2003 |
| | | | WO | 9824933 | A1 | 11-06-1998 |
| WO 0031298 | A1 | 02-06-2000 | AU | 1479200 | A | 13-06-2000 |
| | | | US | 2002048752 | A1 | 25-04-2002 |
| | | | WO | 0031298 | A1 | 02-06-2000 |
| WO 2011035801 | A1 | 31-03-2011 | EP | 2473939 | A1 | 11-07-2012 |
| | | | JP | 5744875 | B2 | 08-07-2015 |
| | | | JP | 2013504104 | A | 04-02-2013 |
| | | | US | 2012165222 | A1 | 28-06-2012 |
| | | | US | 2017039317 | A1 | 09-02-2017 |
| | | | WO | 2011035801 | A1 | 31-03-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014096126 A
- US 20030143584 A
- WO 2011035801 A

**Non-patent literature cited in the description**

- **HOOYBERGHS et al.** *Nucleic Acids Res.,* 2009, vol. 37, e53
- **ONO, N. et al.** *Bioinformatics,* 2008, vol. 24, 1278-1285
- **BURDEN, C. J. ; BINDER, H.** *Phys. Biol.,* 2009, vol. 7, 016004
- **BLOOMFIELD VA et al.** Nucleic Acids Structures, Properties and Functions. University Science Books, 2000
- **HOOYBERGHS, JEF et al.** *Phys. Rev.,* 2010, vol. E 81, 012901
- **HOOYBERGHS, J. et al.** *Nucleic Acids Res.,* 2009, vol. 37, e53
- **HOOYBERGHS, J. et al.** *Biosens. Bioelectron.,* 2010, vol. 26, 1692-1695